(19) Europäisches Patentamt
European Patent Office
Office européen des brevets

(11) EP 4 122 942 A1

(12) **EUROPEAN PATENT APPLICATION**
published in accordance with Art. 153(4) EPC

(43) Date of publication:
**25.01.2023 Bulletin 2023/04**

(21) Application number: **21772632.2**

(22) Date of filing: **19.03.2021**

(51) International Patent Classification (IPC):
***C07H 15/04*** *(2006.01)* ***A61K 31/7028*** *(2006.01)*
***A61P 17/00*** *(2006.01)* ***A61P 17/16*** *(2006.01)*
***A61P 29/00*** *(2006.01)* ***A61K 8/60*** *(2006.01)*
***A61Q 17/04*** *(2006.01)* ***A61Q 19/08*** *(2006.01)*
***A23L 33/10*** *(2016.01)*

(52) Cooperative Patent Classification (CPC):
**A23L 33/10; A61K 8/60; A61K 31/7028; A61P 17/00; A61P 17/16; A61P 29/00; A61Q 17/04; A61Q 19/08; C07H 15/04**

(86) International application number:
**PCT/KR2021/003463**

(87) International publication number:
**WO 2021/187959 (23.09.2021 Gazette 2021/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2020 KR 20200034073**

(71) Applicant: **Athena Co., Ltd.**
**Gyeonggi-do 16226 (KR)**

(72) Inventors:
- **PARK, Eunkyung**
  **Yongin-si, Gyeonggi-do 16849 (KR)**
- **WOO, Dongjin**
  **Yongin-si, Gyeonggi-do 16849 (KR)**

(74) Representative: **Eisenführ Speiser**
**Patentanwälte Rechtsanwälte PartGmbB**
**Postfach 10 60 78**
**28060 Bremen (DE)**

# (54) GLYCEROL GLUCOSIDE-BASED COMPOUND AND COMPOSITION COMPRISING SAME FOR ULTRAVIOLET LIGHT SHIELD OR ANTI-INFLAMMATION

(57) The present invention provides a novel glycerol glucoside-based compound having excellent ultraviolet light shielding performance and anti-inflammatory effect, and a composition including the compound for ultraviolet light shielding or anti-inflammation.

FIG. 1

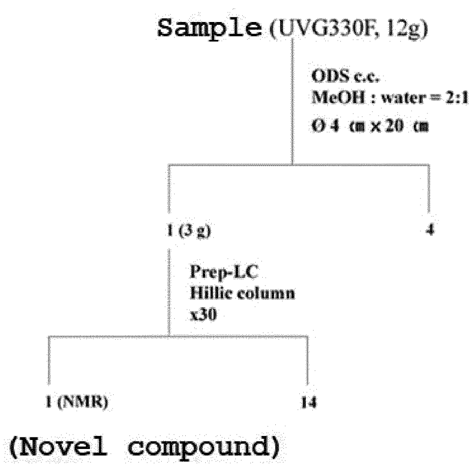




Processed by Luminess, 75001 PARIS (FR)

## Description

TECHNICAL FIELD

The present invention relates to a novel glycerol glucoside (GG)-based compound having excellent ultraviolet light shielding performance, and anti-inflammatory effects, and a composition including the compound for ultraviolet light shielding, antioxidant and anti-inflammation.

BACKGROUND ART

Polysaccharides refer to high molecular weight carbohydrates in which numerous monosaccharides or polysaccharide derivatives are linked together by a glucosidic bond. Most polysaccharides are hydrophilic hydrocolloids and have properties that dissolve or spread in water, so they are widely used in food, pharmaceuticals, and cosmetics.

DETAILED DESCRIPTION OF THE INVENTION

TECHNICAL OBJECTIVES

The present inventors have isolated and purified a novel glycerol glucoside (GG)-based compound having a predetermined chemical structure from natural products such as microalgae or seaweed, and the present invention has been completed by finding that this novel compound has excellent UV shielding, anti-aging, and anti-inflammatory effects.
Accordingly, the present invention is directed to providing the novel compound.
Another technical objective of the present invention is to provide a pharmaceutical composition, a cosmetic composition, or a food composition for UV shield, anti-aging, or anti-inflammation, including the novel compound as an active ingredient.
Other objectives and advantages of the present invention may be more clearly explained by the following detailed description and claims.

TECHNICAL SOLUTION TO THE PROBLEM

In order to achieve the above technical objectives, the present invention provides a compound represented by the following Chemical Formula 1, a pharmaceutically acceptable salt of the compound, or a solvate of the compound:

[Chemical Formula 1]

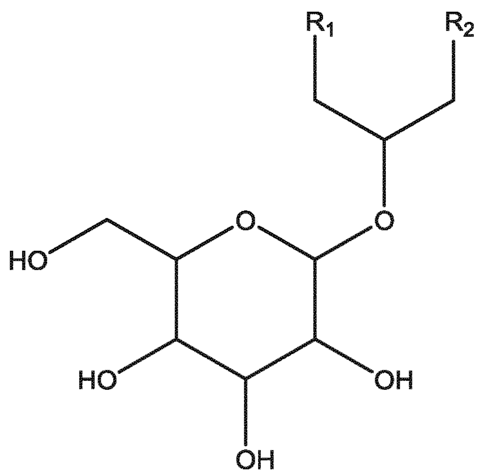

wherein in the above Chemical Formula,
$R_1$ and $R_2$ are the same as or different from each other, each independently being selected from: a hydroxyl group and a $C_1$ to $C_6$ alkoxy group, wherein at least one of $R_1$ and $R_2$ is a $C_1$ to $C_6$ alkoxy group.
According to an embodiment of the present invention, each of $R_1$, $R_2$, or $R_1$ and $R_2$ may be a methoxy group.
The present invention also provides a pharmaceutical composition for UV shield, anti-aging or anti-inflammation, including the compound represented by Chemical Formula 1, the pharmaceutically acceptable salt of the compound, or

the solvate of the compound.

The present invention also provides a cosmetic composition for UV shield, anti-aging, or anti-inflammation, including the compound represented by Chemical Formula 1, the pharmaceutically acceptable salt of the compound, or the solvate of the compound.

The present invention also provides a food composition for UV shield, anti-aging, or anti-inflammation, including the compound represented by Chemical Formula 1, the pharmaceutically acceptable salt of the compound, or the solvate of the compound.

According to an embodiment of the present invention, the food composition may be a functional food, a health supplement or a health functional food.

## EFFECTS OF THE INVENTION

According to an embodiment of the present invention, the compound represented by the above Chemical Formula 1 is a novel glycerol glucoside-based compound isolated and purified for the first time in nature, and is applicable to a composition for UV shield, antioxidant, and/or anti-inflammatory, since it may exhibit excellent UV shielding, anti-aging and anti-inflammatory effects and it is not toxic.

Accordingly, in the present invention, the aforementioned novel glycerol glucoside-based compound may be usefully used as an active ingredient in a composition such as pharmaceuticals, cosmetics, processed food, functional food, food additive, functional beverage or beverage additive for prevention and treatment of UV shield, anti-aging, and/or inflammation-related diseases, allergic diseases, and the like.

The effect according to the present invention is not limited by the description exemplified above, and more various effects are included in the present specification.

## BRIEF DESCRIPTION OF DRAWINGS

FIG. 1 is a schematic diagram illustrating a separation process of a glycerol glucoside-based compound according to an embodiment of the present invention.

FIG. 2 is a $^{1}H$-NMR spectrum analysis result of a glycerol glucoside-based compound according to an embodiment of the present invention.

FIG. 3 is a $^{13}C$-NMR spectrum analysis result of a glycerol glucoside-based compound according to an embodiment of the present invention.

FIG. 4 is a chromatogram analysis result of a glycerol glucoside-based compound according to an embodiment of the present invention.

FIG. 5 is a MASS spectrum analysis result of a glycerol glucoside-based compound according to an embodiment of the present invention.

FIG. 6 is a schematic diagram illustrating signal pathways of p-IKK, p-IκB and p-NF-κB.

FIG. 7 is a photograph illustrating protein bands of p-IKK, p-IκB and p-NF-κB.

## MODE FOR IMPLEMENTATION OF THE INVENTION

Hereinafter, the present invention will be described in detail.

All terms (including technical and scientific terms) used in this specification may be used in the meaning commonly understood by those of ordinary skill in the art to which the present invention pertains, unless otherwise defined. In addition, terms defined in a commonly used dictionary are not to be interpreted ideally or excessively unless clearly defined in particular.

In addition, throughout this specification, when a part "comprises (includes)" a certain component, it means that other components may be further included, rather than excluding other components, unless specifically stated to the contrary. In addition, throughout the specification, "on" or "above" means not only when it is located above or below the target part, but also includes the case where there is another part in the middle, and it does not mean that it is necessarily positioned above the reference in the direction of gravity.

An example of the present invention relates to a compound represented by the following Chemical Formula 1, a pharmaceutically acceptable salt of the compound, or a solvate of the compound.

[Chemical Formula 1]

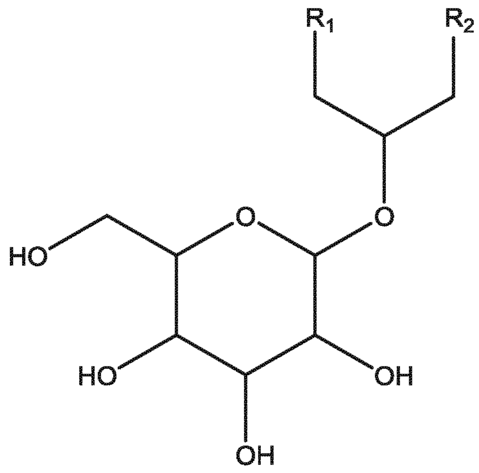

In the above Chemical Formula,
$R_1$ and $R_2$ are the same as or different from each other, and are each independently selected from: a hydroxyl group and a $C_1$ to $C_6$ alkoxy group, where at least one of $R_1$ and $R_2$ is an alkoxy group.

The compound represented by Chemical Formula 1 is a novel glycerol glucoside (GG)-based compound in which glycerol and glucose are conjugated, and may be included in the category of glycerol glucide-based derivatives in which hydrogen at the carbon 1 position in the hydroxyl group is replaced with an organic component. Specifically, the compound of Chemical Formula 1 may have a structure in which at least one alkoxy group (e.g., $R_1$, $R_2$) is bonded to glycerol glucoside (floridoside), and more specifically, may have a structure in which two alkoxy groups are bonded.

For example, in Chemical Formula 1, each of $R_1$, $R_2$, or $R_1$ and $R_2$ may be a $C_1$ to $C_6$ alkoxy group, and more specifically, it may preferably be a methoxy group (methoxy, $-OCH_3$).

As a preferred example, the compound of Chemical Formula 1 may be more specified to a compound represented by Chemical Formula 2 or Chemical Formula 3 below. However, the present invention is not limited thereto.

[Chemical Formula 2]

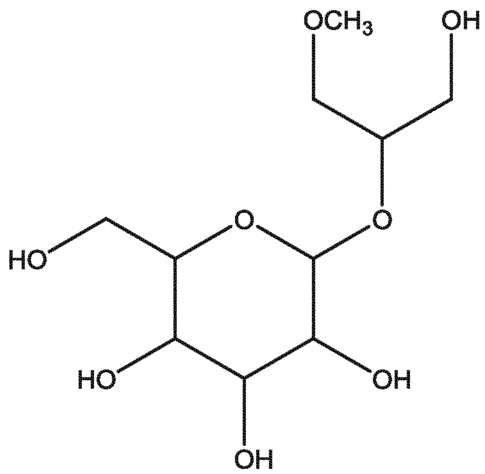

[Chemical Formula 3]

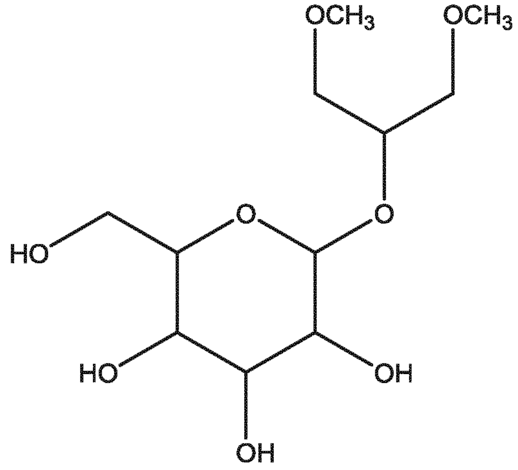

The present invention also provides a salt, preferably a pharmaceutically acceptable salt, of the compound represented by Chemical Formula 1, specifically any one of Chemical Formulas 2 to 3.

As used herein, the "pharmaceutically acceptable salt" means a salt suitable for use in contact with human and lower animal tissues without causing excessive toxicity, irritation, allergic reaction, and the like within the scope of pure medical judgment. The pharmaceutically acceptable salts are well known in the art and are described in detail in, for example, the document "S.M. Berge et al., J. Parmaceutical Sciences, 66, 1, 1977." The salt may be prepared in situ during final isolation and purification of the compound of the present invention, or may be prepared by separately reacting with an inorganic base or an organic base. Preferred examples of the base addition salt form may include ammonium salts; alkali salts and alkaline earth metal salts such as salts of lithium, sodium, potassium, magnesium, calcium, and the like; salts with organic bases, for example, primary, secondary and tertiary aliphatic and aromatic amines such as methylamine, ethylamine, propylamine, isopropylamine, four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; benzathine, N-methyl-D-glucamine, 2-amino-2-(hydroxymethyl)-1,3-propanediol, hydrabamine salt; and salts with amino acids such as arginine and lysine.

In addition, the present invention may include a hydrate or solvate of the compound represented by Chemical Formula 1, and derivatives thereof. Among the solvates, a solvent is not particularly limited, and may include all conventional solvents known in the art.

According to the present invention, the compound represented by Chemical Formula 1 may be isolated from a natural product or prepared according to a chemical synthesis method known in the art.

For example, the compound of Chemical Formula 1 may be isolated and purified from natural products, such as natural plants, microalgae, seaweed, and the like. That is, it may be obtained from a portion of a natural product using a conventional method of extracting and separating the material. These raw materials may be appropriately dried and macerated in order to obtain a desired extract, or the raw materials may be dried and then an appropriate solvent, for example, water such as purified water, or an organic solvent having 1 to 6 carbon atoms may be used. Non-limiting examples of the applicable organic solvent may include lower alcohols having 1 to 4 carbon atoms such as methanol, ethanol, propanol, isopropanol, and butanol; and various solvents such as acetone, ether, chloroform, ethyl acetate, methylene chloride, hexane, cyclohexane, and petroleum ether which may be used alone or in combination.

The natural products that may be used in the present invention are not particularly limited, and may be, for example, conventional natural plants, microalgae, seaweeds, or a combination thereof known in the art. Specifically, it may be an algae such as codium fragile (Sea staghorn), hizikia fusiforme (Seaweed fusiforme), and agarophyton vermiculophyllum (Sea string) or a microalgae such as chlorella, and more specifically, it may be gracilaria spp. native to Korea, Japan, and Taiwan.

As the extraction method used in the present invention, any method commonly used for extraction of plants, microalgae, algae or herbal medicines may be used without limitation; for example, cold extraction, room temperature extraction, hot water extraction, ultrasonic extraction, percolation method or reflux cooling extraction method may be used, but the present invention is not limited thereto. The extraction process may be carried out once, but may be repeated two or more times if necessary. As an example of the extraction method, 2700 to 3100 g of purified water, specifically about 2,970 g (approximately 1%) of purified water is added to 20 to 40 g of raw material, specifically about 30 g of raw material, followed by hot water extraction at 87 to 95°C, specifically at 90°C, and when a temperature of the

purified water reaches 85 to 95°C, the raw material is added to start the extraction, and a sample may be obtained every predetermined time while the extraction proceeds for about 7 to 10 hours.

The desired extract may be further subjected to a process such as fractionation to separate the compound represented by Chemical Formula 1 of the present invention, and a fractionation solvent used in this case may use the aforementioned extraction solvent without limitation. Purification may also be performed using purification methods known to those skilled in the art to which the present invention pertains. In non-limiting examples of such purification methods, separation and purification may be performed by gradient chromatography in which reverse phase partition chromatography, normal phase adsorption chromatography, ion exchange chromatography, size exclusion chromatography, or an additional purification method consisting of a combination of one or more thereof may be performed. As the chromatography, column chromatography filled with various synthetic resins such as silica gel or activated alumina and high-performance liquid chromatography (HPLC) may be used alone or in combination. However, the method for extraction and separation and purification of the compound is not necessarily limited to the above method.

The compound represented by the above Chemical Formula 1, isomers, pharmaceutically acceptable salts or solvates thereof are well soluble in water, stable to heat and light, and non-toxic, thus exhibiting no irritation to the skin and being effective in blocking UV rays due to its ability to absorb UV light. In addition, since it has antioxidant and/or anti-inflammatory effects, it may be usefully utilized as a functional composition for UV shield, antioxidant, and/or anti-inflammatory.

Another example of the present invention relates to a pharmaceutical composition for UV shield, anti-aging, or anti-inflammation, including, as an active ingredient, the compound represented by Chemical Formula 1, or the pharmaceutically acceptable salt or the solvate thereof.

As used herein, the term "active ingredient" refers to a component capable of exhibiting the desired activity by itself or in combination with a carrier that is not active by itself.

As used herein, "anti-inflammatory" is meant to include alleviation (relief of symptoms), treatment, and suppression or delay of the onset of inflammatory diseases as defined below. The "inflammatory disease" is a pathological symptom caused by an inflammatory reaction specified by an external physical or chemical stimulus or an infection of an external infectious agent such as bacteria, mold, virus, or various allergens, or a local or systemic biological defense response to autoimmunity. Such an inflammatory response may involve a series of complex physiological reactions such as activation of enzymes (e.g., iNOS, COX-2, etc.) related to various inflammatory mediators and immune cells, secretion of inflammatory mediators (e.g., secretion of NO, TNF-$\alpha$, IL-6, etc.), infiltration of body fluids, cell migration and tissue destruction, and may appear externally by symptoms such as erythema, pain, edema, fever, and deterioration or loss of specific functions of the body.

An amount of the compound of Chemical Formula 1 used as an active ingredient in the pharmaceutical composition according to the present invention may be appropriately adjusted according to the form and purpose of use, the patient's condition, the type and severity of symptoms, and the like. For example, a content of the compound of Chemical Formula 1 may be in a range of 0.000001 to 50 wt%, and preferably in a range of 0.0001 to 40 wt%, with respect to the total weight of a solid content. However, this may be increased or decreased according to the needs of the administrator, and may be appropriately increased or decreased according to various factors such as diet and nutritional status, so it is not limited to the above range.

The pharmaceutical composition according to the present invention may further include an adjuvant such as a pharmaceutically suitable and physiologically acceptable carrier, excipient and diluent, in addition to the compound of Chemical Formula 1, or the pharmaceutically acceptable salt or the solvate thereof. For example, in the case of a formulation for oral administration, it may be formulated using an excipient, a binder, a disintegrant, a lubricant, a solubilizer, a suspending agent, a preservative, an extender, or the like.

The pharmaceutical composition of the present invention may be administered to mammals including humans by various routes. A mode of administration may be any method commonly used, for example, it may be administered by a route such as orally or parenterally (e.g., skin, intravenous, intramuscular, subcutaneous), and preferably orally.

A dosage of the pharmaceutical composition of the present invention may be determined by an expert according to various factors such as the patient's condition, age, weight, degree of cartilage damage, and disease progression. In addition, a daily dose of the pharmaceutical composition or a dose of 1/2, 1/3, or 1/4 thereof may be contained per unit dosage form, and may be administered 1 to 6 times a day. However, the present invention is not particularly limited thereto.

Another example of the present invention relates to a cosmetic composition for UV shield, anti-aging, or anti-inflammation improvement including, as an active ingredient, the compound represented by Chemical Formula 1, or the pharmaceutically acceptable salt or the solvate thereof.

A content of the compound of Chemical Formula 1 used as an active ingredient in the cosmetic composition according to the present invention is not particularly limited, and may be appropriately adjusted depending on the type and purpose of use, skin condition, type and severity of symptoms, and the like.

The cosmetic composition according to the present invention may include ingredients commonly used in cosmetic compositions in addition to the compound of Chemical Formula 1, or the pharmaceutically acceptable salt or the

solvate thereof. For example, it may include, without limitation, common adjuvants and carriers known in the art such as stabilizers, solubilizers, vitamins, pigments and fragrances.

The cosmetic composition of the present invention may be used in various ways, such as cosmetics and face washes for UV shield, antioxidant, anti-inflammatory, and anti-wrinkle effects of the skin. Products to which the present composition may be added may include, for example, cosmetics such as various creams, lotions, and skins, and cleansing agents, face washes, soaps, treatments, and cosmetic liquids. The cosmetic composition of the present invention may include a composition selected from: watersoluble vitamins, oil-soluble vitamins, polymer peptides, polymer polysaccharides, sphingolipids, and seaweed extract.

In addition, the cosmetic composition of the present invention may be prepared in any formulation conventionally prepared in the art. For example, it may take the form of a solution, emulsion, viscous mixture, and the like, and more specifically, it may be formulated as, for example, solution, suspension, emulsion, paste, gel, cream, lotion, powder, soap, surfactant-containing cleansing, oil, powder foundation, emulsion foundation, wax, foundation, spray, pack, cosmetic liquid, hair cosmetic, and the like. However, the present invention is not limited thereto.

Another example of the present invention is a food composition for UV shield, anti-aging, or anti-inflammation improvement including, as an active ingredient, the compound represented by the following Chemical Formula 1, the pharmaceutically acceptable salt or the solvate thereof.

As used herein, 'food' is not limited to health supplements, health functional foods, functional foods, and the like, and it may also refer to natural foods, processed foods, and general food materials added with the compound of Chemical Formula 1, the isomer thereof, the pharmaceutically acceptable salt or the solvate thereof.

In addition, the 'health functional food' refers to a food including, as raw materials, certain ingredients with useful functions for the human body for the purpose of health supplementation, or manufactured and processed by methods such as extracting, concentrating, refining, or mixing a specific ingredient in food raw material. In addition, 'functional food' refers to food designed and processed to sufficiently exert biological control functions of food ingredients, such as biological defense, regulation of biological rhythm, prevention and recovery of disease, and it also has functions related to disease prevention and disease recovery.

The food composition according to the present invention may be added with the composition as it is or used together with other food or food compositions. In this case, an amount of the active ingredient used may be appropriately determined according to the purpose of its use, and is not particularly limited. In general, the compound of Chemical Formula 1, the isomer, the pharmaceutically acceptable salt or the solvate thereof according to the present invention may be added in an amount in a range from 0.000001 to 50 percent by weight (wt%) with respect to the total weight of the raw material of the food or beverage when preparing the food or beverage.

The food composition of the present invention may include ingredients commonly used in food compositions in the art, in addition to the compound of Chemical Formula 1, the isomer, the pharmaceutically acceptable salt or the solvate thereof. For example, one or more additives selected from: organic acids, phosphates, antioxidants, lactose casein, dextrin, glucose, sugar and sorbitol may be further included. In addition, it may further include various nutrients, vitamins, minerals (electrolytes), flavoring agents such as synthetic flavoring agents and natural flavoring agents, coloring agents and thickening agents, pectic acid and its salts, alginic acid and its salts, organic acids, protective colloidal thickeners, pH adjusting agents, stabilizers, preservatives, glycerin, alcohols, carbonation agents used in carbonated beverages, and the like.

The food composition of the present invention may be prepared in any dosage form conventionally prepared in the art, and for example, it is also within the scope of the present invention to be provided in the form of tablets, granules, pills, capsules, liquid preparations, syrups or beverages.

In the food composition according to the present invention, the type of food to which the compound of Chemical Formula 1, the isomer, the pharmaceutically acceptable salt or the solvate thereof may be added is not particularly limited. Examples thereof may include various foods, powders, granules, tablets, capsules, syrups, beverages, gums, tea, vitamin complexes, health functional foods, and the like. More specific examples may include meat, sausage, bread, chocolate, candy, snacks, confectionery, pizza, ramen, other noodles, gums, dairy products including ice cream, various soups, beverages, tea, drinks, alcoholic beverages and vitamin complexes, and other nutritional supplements, but are not limited to these types of foods.

Hereinafter, the present invention will be described in more detail through examples. These examples are only for illustrating the present invention in more detail, and it will be apparent to those of ordinary skill in the art that the scope of the present invention is not limited by these examples according to the gist of the present invention.

### [Example: Preparation and Structural Analysis of Samples]

### 1-1: Materials and Reagents

A powder sample of seaweed extract (UVG330F) was diluted with distilled water. The obtained material was

sequentially separated and purified with HPLC and Preparative-LC as illustrated in FIG. 1, and thus samples were obtained.

In order to analyze the chemical structure of the obtained sample, structural analysis was performed using the following materials and reagents. In such a case, all compounds used for structural analysis of the compound were purchased and used from Sigma Chemical Co., Ltd. (St. Louis, MO, USA).

Preparative-liquid chromatography (prep-LC) (LC-Forte/R, YMC, Japan)
Venusil Hilic (21.2 × 250 mm, 5 μm, Agela, USA)
VisionHT Hilic (4.6 × 250 mm, 5 μm, Grace, USA)
Kiesel gel 60F254 plate (Merck Co., Darmastdt, Germany)
RP-18 F254s plates (Merck Co., Darmastdt, Germany)
Bruker AVANCE II 400 (1H NMR at 400 MHz, 13C NMR at 100 MHz)
6530 Accurate-Mass Triple quald.
LC-MS/MS (Agilent Technologies, Germany)

More specifically, the separation conditions and analysis conditions of the sample are shown in Tables 1 and 2 below.

[Table 1]

| | |
|---|---|
| Separation conditions | preparative-LC (YMC-forte) |
| Column | Venusil Hilic (21.2 X 250 mm, 5 □m, Agela, USA) |
| Flow rate | 10 ml/min |
| Injection vol. | 1 Ml |
| Column temp. | 30℃ |
| Mobile phase | A; 0.1% Formic acid in Water, B; 0.1% Formic acid in Acetonitrile gradient |
| Detector | UV, 254 nm |
| Gradient | (see gradient table below) |

| | time(min.) | B% |
|---|---|---|
| 1 | 0 | 60 |
| 2 | 20 | 60 |

[Table 2]

| | |
|---|---|
| Analysis conditions | Agilent Technologies 6410 Triple Quad (LC-MS/MS) |
| Column | VisionHT-Hillic (4.6 mm × 250 mm, 5.0 µm) |
| Flow rate | 0.4 ml/min |
| Injection vol. | 10 µℓ |
| Column temp. | 30℃ |

<table>
<tr><td>Mobile phase</td><td>A; 0.1% Formic acid in Water, B; 0.1% Formic acid in Acetonitrile gradient</td></tr>
<tr><td>MS condition</td><td><table><tr><td>Ionization Mode</td><td>-ESI, scan mode</td></tr><tr><td>Gas temp.</td><td>350 ℃</td></tr><tr><td>Capillary volt.</td><td>4000 V</td></tr><tr><td>Nebulizer</td><td>40 psig</td></tr><tr><td>Fragmentor</td><td>135 V</td></tr></table></td></tr>
<tr><td>Gradient</td><td><table><tr><td></td><td>time(min.)</td><td>B%</td></tr><tr><td>1</td><td>0</td><td>60</td></tr><tr><td>2</td><td>15</td><td>60</td></tr></table></td></tr>
</table>

**1-2: Structural analysis of compounds**

In order to identify the structure of the compound isolated and purified in Example 1-1, structural analysis was performed in the following manner.

1) A chemical environment related to hydrogen was analyzed from a chemical shift on a $^1$H-NMR spectrum.
2) A three-dimensional arrangement with adjacent hydrogens was analyzed from a coupling constant, and the number of hydrogens was identified from the integration.
3) The number of carbons constituting the compound and a surrounding environment of carbon were analyzed from a $^{13}$C-NMR spectrum.
4) A multiplicity of carbon was identified from DEPT NMR data.
5) An environment of adjacent hydrogen was analyzed from a $^1$H-$^1$H COSY spectrum and a partial structure was identified.
6) An overall chemical structure including a partial structure was identified from a HMBC spectrum.
7) A spatial and stereoscopic relative structure was analyzed from a NOE spectrum.
8) A more definite relative three-dimensional structure was identified from a NOESY spectrum.

**1-2-1: NMR structure analysis**

As may be appreciated in FIG. 2, in the $^1$H-NMR spectrum, an anomer proton signal δH 5.05 (1H, d, J = 3.6

Hz, H-1) derived from sugar, an oxygenated methine proton signal seen as a sugar moiety, and an oxygenated methylene proton signal δH 3.63-4.00 (5H, m, H-2, 3, 4, 5, and 6) were observed to confirm the presence of glucose or galactose. In addition, it may be identified that two methoxy groups exist through δH 3.86 (3H, t, J = 6.4 Hz, H-$OCH_3$) and δH 3.05 (3H, t, J = 6.4 Hz, H-$OCH_3$) signals.

In addition, as may be appreciated in FIG. 3, the total number of carbons in the $^{13}$C-NMR spectrum was 11, through which it was confirmed that glycerol composed of three carbons and two methoxy groups, including one sugar molecule, were present. In addition, it was confirmed that the sugar was galactose based on an anomer carbon signal $\delta_c$ 98.01 (C-1) derived from sugar, an oxygenated methine carbon signal [$\delta_c$ 71.05 (C-5), $\delta_c$ 69.30 (C-3), $\delta_c$ 69.23 (C-4), and $\delta_c$ 68.43 ( C-2)], and an oxygenated methylene carbon signal $\delta_c$ 61.12 (C-6).

The presence of glycerol was confirmed by one oxygenated methine carbon signal $\delta_c$ 78.66 (C-2') and two oxygenated methylene carbon signals [$\delta_c$ 61.36 (C-1') and 60.30 (C-3')], and it was confirmed that two molecules of -$OCH_3$ were bound to glycerol through a coupling pattern of the methoxy group.

Through this, it was confirmed that the compound (sample) had a novel structure in which two methoxy molecules were bonded to 2-O-α-D-galactopyanosylglycerol (floridoside) (see Chemical Formula 3 below).

[Chemical Formula 3]

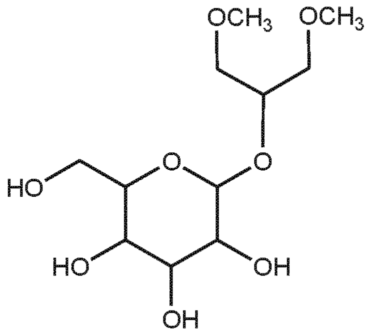

Chemical Formula: $C_{11}H_{22}O_8$
Exact Mass: 282.13

1-2-2: Mass structure analysis

ESI-MS was measured to identify a molecular weight of the compound (sample), and as a result, 267 [M-$OCH_3$]$^-$ and 327 [M+formic acid-H]$^-$ were identified in the negative mode, and thus it was confirmed that the molecular weight was 282 (see FIG. 5 below).

**[Experimental Example 1: UV shield evaluation (in *vitro* ultraviolet UV-A, UV-B evaluation test)]**

For a glycerol glucoside extract powder of the present invention extracted and fractionated from seaweed, an estimation test was performed using a UV spectrophotometer as a preliminary step of a human application test.

For the sample, different seaweed extract powders P and G were diluted in distilled water to obtain concentrations of 50%, 25%, and 12.5%, respectively. Each prepared sample was spread on a PMMA plate to 1.3mg/cm$^2$, dried for 15 minutes, put in a SPF-290AS™ SPF testing analyzer system, and measurement was made at 8 points on the plate at a UV absorbance in a range of 290 to 400nm; where 3 plates were used for each sample to calculate an average value and a deviation by repeated measurement.

[Table 3]

| | Powder P | SPF (UV-B) | PA (UV-A) |
|---|---|---|---|
| Sample Concentration 50% | Sample 1 | 15.6 | 25.9 |
| | Sample 2 | 13.6 | 22.4 |
| | Sample 3 | 15.9 | 25.6 |
| | Aver. | 15.0 | 24.7 |
| | Deviation (SD) | 1.2 | 2.0 |

(continued)

| | Powder P | SPF (UV-B) | PA (UV-A) |
|---|---|---|---|
| Sample Concentration 25% | Sample 1 | 3.0 | 2.3 |
| | Sample 2 | 3.5 | 2.7 |
| | Sample 3 | 2.9 | 2.3 |
| | Aver. | 3.1 | 2.4 |
| | Deviation (SD) | 0.3 | 0.3 |
| Sample Concentration 12.5% | Sample 1 | 1.6 | 1.3 |
| | Sample 2 | 1.7 | 1.4 |
| | Sample 3 | 1.6 | 1.3 |
| | Aver. | 1.6 | 1.3 |
| | Deviation (SD) | 0.1 | 0.0 |

[Table 4]

| | Powder G | SPF (UV-B) | PA (UV-A) |
|---|---|---|---|
| Sample Concentration 500 | Sample 1 | 5.3 | 4.1 |
| | Sample 2 | 5.7 | 4.5 |
| | Sample 3 | 5.6 | 4.5 |
| | Aver. | 5.6 | 4.4 |
| | Deviation (SD) | 0.2 | 0.2 |
| Sample Concentration | Sample 1 | 3.4 | 2.8 |
| | Sample 2 | 3.7 | 3.2 |
| 25% | Sample 3 | 3.4 | 2.9 |
| | Aver. | 3.5 | 3.0 |
| | Deviation (SD) | 0.1 | 0.2 |
| Sample Concentration 12.5% | Sample 1 | 2.3 | 2.1 |
| | Sample 2 | 2.3 | 1.9 |
| | Sample 3 | 2.5 | 2.0 |
| | Aver. | 2.4 | 2.0 |
| | Deviation (SD) | 0.1 | 0.1 |

As shown in Tables 3 and 4 above, the glycerol glucoside extract powder of the present invention extracted and fractionated from seaweed exhibited an excellent UV shield effect, and in particular, it was found that the SPF (UV-B) value tends to be significantly high.

**[Experimental Example 2: Anti-inflammatory evaluation]**

In order to identify the anti-inflammatory effect of the glycerol glucoside-based compound, the following experiments were performed, respectively.

**3-1. Cell viability measurement using MTT assay**

In order to evaluate a cell viability of the glycerol glucoside-based compound sample (EPS-S), an MTT assay

was performed.

Specifically, subcultured RAW264.7 cells and HDF cells were aliquoted into a 96-well plate at $1\times10^5$, respectively, and cultured for 24 hours at 37°C in an incubator containing 5% carbon dioxide, and then, a culture media was replaced with a serum-free media. Then, each sample was treated onto each cell by concentration. After culturing for 24 hours, an MTT solution was put into each well, reacted for 2 hours under light blocking, a supernatant was removed, a formed formazan was completely dissolved in DMSO, and an absorbance was measured at 540 nm with a microplate reader. In such a case, SDS was used as a positive control group. The test result value was calculated as in Equation 1 below and expressed as the cell viability compared to an untreated group, and was expressed as a mean ± standard deviation.

[Equation 1]

$$Cell\ viability(\%) = \frac{Absorbance\ of\ treated\ sample}{Absorbance\ of\ untreated\ sample} \times 100$$

[Table 5]

| Mean (%) ± standard deviation of cell viability for each concentration of sample | | | | |
|---|---|---|---|---|
| Sample/Concentrati on | 1 ppm | 10 ppm | 100 ppm | 1,000 ppm |
| Ex. (EPS-S) | 103.36±0.8 8 | 104.4713.6 1 | 108.58±1.9 9 | 113.79±0.1 3 |
| Comp. Ex. (SDS) | 99.95±0.72 | 96.29±0.16 | 7.75±0.14 | 6.67±0.16 |

As shown in Table 5, the cell viability of the glycerol glucoside-based compound sample (EPS-S) obtained in the present Example for RAW264.7 cells was 103.36% at 1 ppm, 104.47% at 10 ppm, 108.58% at 100 ppm, and 113.79% at 1000 ppm.

On the other hand, it was found that the cell viability of the SDS sample as a positive control group for RAW264.7 cells was 99.95% at 1 ppm, 96.29% at 10 ppm, 7.75% at 100 ppm, and 6.67% at 1,000 ppm.

**3-2. Analysis of nitric oxide (NO) production inhibition ability (Non-enzymatic Method)**

An NO assay was performed to evaluate the nitric oxide (NO) production inhibition ability of the glycerol glucoside-based compound sample (EPS-S).

Specifically, subcultured RAW264.7 cells were aliquoted in a 96-well plate at $1\times10^5$ cells/well, and cultured for 24 hours at 37°C in an incubator containing 5% carbon dioxide, and then, a culture media was replaced with a serum-free media. Then, each sample was diluted by concentration, treated onto the cell with LPS (final 1 μg/mL), and after culturing for 24 hours, the cell culture medium and a Griess reagent were reacted, and an absorbance was then measured at 540 nm with a microplate reader. Dexamethasone was used as a positive control group. After the test was conducted once, each absorbance value was substituted into Equation 2 below to obtain the NO production inhibition rate (%), and it was expressed as the mean ± standard deviation.

[Equation 2]

$$NO\ production\ inhibition\ rate(\%) = 100 - \frac{Absorbance\ of\ treated\ sample - Absorbance\ of\ untreated\ sample}{Absorbance\ of\ negative\ control - Absorbance\ of\ untreated\ sample} \times 100$$

[Table 6]

| Mean (%) ± standard deviation of NO production inhibition rate for each concentration of sample | | | | |
|---|---|---|---|---|
| Sample/Concentration of Ex. | 1 ppm | 10 ppm | 100 ppm | 1,000 ppm |
| Ex. (EPS-S) | -5.66±2.00 | 6.10±3.60 | 14.16±2.95 | 30.28±1.64 |
| Sample/Concentration of Comp. Ex. | 10 μM | 20 μM | 40 μM | 80 μM |
| Comp. Ex. (Dexamethasone) | 15.57±2.01 | 17.98±3.11 | 27.63±1.74 | 33.77±2.31 |

As shown in Table 6, the NO production inhibition rate of the glycerol glucoside-based compound sample (EPS-S) obtained in the present Example was -5.66% at 1 ppm, 6.10% at 10 ppm, 14.16% at 100 ppm, and 30.28% at 1000 ppm, respectively.

On the other hand, it was found that the NO production inhibition rate of dexamethasone (the positive control group) was 15.57% at 10 μM, 17.98% at 20 μM, 27.63% at 40 μM, and 33.77% at 80 μM.

**3-3. Analysis of TNF-α production inhibition ability using ELISA assay**

A TNF-α production inhibition ability of the sample in RAW264.7 cells was evaluated using a TNF-α ELISA assay.

Specifically, subcultured RAW264.7 cells were aliquoted into a 6-well dish at $1.5\times10^6$ cells/well, and cultured for 24 hours at 37°C in an incubator containing 5% carbon dioxide, and then a culture media was replaced with a serum-free media. Then, each sample was diluted by concentration and treated onto the cells with LPS (final 10 ng/mL). After culturing for 24 hours, the cell culture medium was recovered and the TNF-α ELISA assay was performed.

50 μL of an assay diluent was put into each well, and 50 μL of each of a standard solution and a cell culture solution were added to each well and reacted at room temperature for 2 hours. Thereafter, the reaction solution was removed from each well, washed with a wash buffer, 100 μL of TNF-α conjugate was added, and after reacting at room temperature for 2 hours, the reaction solution was removed from each well and washing is performed with a wash buffer.

Thereafter, 100 μL of a substrate solution was added and a reaction under light blocking was carried out at room temperature, and then 100 μL of a stop solution was added, and an absorbance was measured at 450 and 570 nm. Dexamethasone was used as a positive control group. After the test was conducted once, a TNF-α synthesis amount was calculated by substituting the absorbance value into a TNF-α standard solution calibration curve, and then the TNF-α production inhibition rate (%) was calculated using Equation 3 below, and expressed as the mean ± standard deviation of the experiment.

[Equation 3]

$$TNF-\alpha\ production\ inhibition\ rate\ (\%)$$

$$= 100 - \frac{Treated\ sample\ TNF-\alpha\ production\ amount\ -\ Untreated\ group\ TNF-\alpha\ production\ amount}{Negative\ control\ TNF-\alpha\ production\ amount\ -\ Untreated\ group\ TNF-\alpha\ production\ amount} \times 100$$

[Table 7]

| TNF-α production inhibition ability (%) ± standard deviation for each concentration of the sample | | | | | |
|---|---|---|---|---|---|
| Sample/Concentrat ion of Ex. | 10 ppm | 100 ppm | 500 ppm | 1,000 ppm | |
| Ex. (EPS-S) | 29.6911. 06 | 34.9112. 09 | 49.7411. 99 | 77.6112. 05 | |
| Sample/Concentrat ion of Comp. Ex. | 1 μM | 5 μM | 10 μM | 20 μM | 80 μM |
| Comp. Ex. (Dexamethasone) | 13.82±1. 66 | 34.68±1. 07 | 46.47±2. 05 | 52.08±1. 38 | 65.05±0. 60 |

As shown in Table 7, the TNF-α production inhibition rate of the glycerol glucoside-based compound sample (EPS-S) obtained in the present Example was 29.69% at 10 ppm, 34.91° at 100 ppm, 49.74% at 500 ppm, and 77.61%

at 1000 ppm, respectively.

On the other hand, the TNF-α production inhibition rate of dexamethasone (the positive control group) was 13.82% at 1 μM, 34.68% at 5 μM, 46.47° at 10 μM, 52.08% at 20 μM, and 65.05% at 50 μM, respectively.

**2-4. IL-6 production inhibition analysis using ELISA assay**

An IL-6 ELISA assay was used to evaluate an IL-6 production inhibition ability of the sample in RAW264.7 cells.

Specifically, subcultured RAW264.7 cells were aliquoted into a 6-well dish at $1.5\times10^6$ cells/well and cultured for 24 hours at 37°C in an incubator containing 5% carbon dioxide, and then a culture media was replaced with a serum-free media. Then, each sample was diluted by concentration and treated onto the cells with LPS (final 10 ng/mL). After culturing for 24 hours, the cell culture medium was recovered and an IL-6 ELISA assay was performed.

50 μL of an assay diluent was put into each well, and 50 μL of each of a standard solution and a cell culture solution were added to each well and reacted at room temperature for 2 hours. Thereafter, the reaction solution was removed from each well, washed with a wash buffer, 100 μL of IL-6 conjugate was added, and after reaction at room temperature for 2 hours, the reaction solution was removed from each well and washing is performed with a wash buffer.

Next, 100 μL of a substrate solution was added and a reaction under light blocking was carried out at room temperature, and then 100 μL of a stop solution was added, and an absorbance was measured at 450 and 570 nm. Dexamethasone was used as a positive control group. After the test was conducted once, an IL-6 production amount was calculated by substituting the absorbance value into a IL-6 standard solution calibration curve, and then the IL-6 production inhibition rate (%) was calculated using Equation 4 below, and expressed as the mean ± standard deviation of the experiment.

[Equation 4]

$$IL\text{-}6\ production\ inhibition\ rate\ (\%)$$

$$= 100 - \frac{Treated\ sample\ IL\text{-}6\ production\ amount\ -\ Untreated\ group\ IL\text{-}6\ production\ amount}{Negative\ control\ IL\text{-}6\ production\ amount\ -\ Untreated\ group\ IL\text{-}6\ production\ amount} \times 100$$

[Table 8]

| IL-6 production inhibition ability (%) ± standard deviation for each concentration of the sample | | | | | |
|---|---|---|---|---|---|
| Sample/Concentra tion of Ex. | 10 ppm | 100 ppm | 500 ppm | 1,000 ppm | |
| Ex. (EPS-S) | 10.14±0. 69 | 23.7911. 41 | 36.2711. 08 | 92.44±0. 29 | |
| Sample/Concentra tion of Comp. Ex. | 1 μM | 5 μM | 10 Mm | 20 μM | 80 μM |
| Comp. Ex. (Dexamethasone) | 12.7411. 18 | 48.35±2. 64 | 55.97±0. 50 | 63.29±0. 85 | 88.70±0. 27 |

As shown in Table 8, the IL-6 production inhibition rate of the glycerol glucoside-based compound sample (EPS-S) obtained in the present Example was 10.14% at 10 ppm, 23.79° at 100 ppm, 36.27% at 500 ppm, and 92.44% at 1000 ppm, respectively.

On the other hand, it was found that the IL-6 production inhibition rate of dexamethasone (the positive control group) was 12.74% at 1 μM, 48.35% at 5 μM, 55.97% at 10 μM, 63.29% at 20 μM, and 88.70% at 50 μM, respectively.

2-5. Analysis of PGE2 production inhibition ability using **ELISA assay**

A PGE2 production inhibition ability of the sample in RAW264.7 cells was evaluated using a PGE2 ELISA assay.

Specifically, subcultured RAW264.7 cells were aliquoted into a 6-well dish at $1.5\times10^6$ cells/well and cultured for 24 hours at 37°C in an incubator containing 5% carbon dioxide, and then a culture media was replaced with a serum-free media. Then, each sample was diluted by concentration and treated onto the cells with LPS (final 10 ng/mL). After culturing for 24 hours, the cell culture medium was recovered and a PGE2 ELISA assay was performed.

100 μL of each of a standard solution and a cell culture solution were put into each well, and then 50 μL of PGE2 conjugate and 50 μL of PGE2 antibody were sequentially added and reacted at room temperature for 2 hours.

Then, the reaction solution was removed from each well, washing is performed with a wash buffer, 200 μL of a substrate solution was then added, and reacted at room temperature for 45 minutes. Then, 50 μL of a stop solution was added and an absorbance was measured at 405 and 570 nm. Dexamethasone was used as a positive control group. After the test was conducted once, an amount of PGE2 production was calculated by substituting the absorbance value into a PGE2 standard solution calibration curve, and the PGE2 production inhibition rate (%) was calculated using Equation 5 below and expressed as the mean ± standard deviation.

[Equation 5]

$$PGE2\ production\ inhibition\ rate\ (\%) = 100 - \frac{Treated\ sample\ PGE2\ production\ amount\ -\ Untreated\ group\ PGE2\ production\ amount}{Negative\ control\ PGE2\ production\ amount\ -\ Untreated\ group\ PGE2\ production\ amount} \times 100$$

[Table 9]

| PGE2 production inhibitory ability (%) ± standard deviation for each concentration of the sample | | | | | |
|---|---|---|---|---|---|
| Sample/Concentra tion of Ex. | 10 ppm | 100 ppm | 500 ppm | 1,000 ppm | |
| Ex. (EPS-S) | 28.34±0. 00 | 36.50±3. 44 | 47.21±3. 88 | 94.13±0. 20 | |
| Sample/Concentra tion of Comp. Ex. | 1 μM | 5 μM | 10 Mm | 20 μM | 80 μM |
| Comp. Ex. (Dexamethasone) | 62.29±0. 97 | 81.43±0. 23 | 83.55±0. 86 | 86.38±0. 61 | 96.38±0. 13 |

As shown in Table 9, the PGE2 production inhibition rate of the glycerol glucoside-based compound sample (EPS-S) obtained in the present Example was 28.34% at 10 ppm, 36.50% at 100 ppm, 47.21% at 500 ppm, and 94.13% at 1000 ppm, respectively.

On the other hand, the PGE2 production inhibition rate of dexamethasone (the positive control group) was 62.29% at 1 μM, 81.43% at 5 μM, 83.55% at 10 μM, 86.38% at 20 μM, and 96.38% at 80 μM, respectively.

**2-6. COX-2 and iNOS mRNA expression analysis**

An RT-qPCR was performed to evaluate an mRNA expression inhibition ability of COX-2 and iNOS of the glycerol glucoside-based compound sample (EPS-S).

Specifically, subcultured RAW264.7 cells were aliquoted into a 60 mm dish at $1.5\times10^6$ cells, and cultured at 37°C in an incubator containing 5% carbon dioxide for 24 hours, and then a culture media was replaced with a serum-free media. Then, each sample was diluted by concentration and treated onto the cells with LPS (final 100 ng/mL). After culturing for 24 hours, cells were recovered, RNA was extracted, and cDNA was synthesized. For cDNA, qPCR was performed after mixing Real-time PCR Master Mix with TaqMan probes of each of COX-2 and iNOS. Dexamethasone was used as a positive control group. The test was conducted once, and the mRNA expression inhibition rate was obtained by analyzing the result by the ΔΔCt method, and then substituted into Equation 6 below to obtain the mRNA expression inhibition rate of COX-2 and iNOS of the treated sample group compared to the negative control group.

[Equation 6]

$$mRNA\ expression\ inhibition\ rate\ (\%) = \frac{Treated\ sample\ mRNA\ expression\ amount}{Negative\ control\ mRNA\ expression\ amount} \times 100$$

[Table 10]

| COX-2 mRNA expression inhibition rate (%) ± standard deviation for each concentration of sample | | | | | |
|---|---|---|---|---|---|
| Sample/Concentra tion of Ex. | 10 ppm | 100 ppm | 500 ppm | 1,000 ppm | |
| Ex. (EPS-S) | 9.09±3.1 6 | 74.44±0. 91 | 76.43±1. 11 | 83.19±1. 30 | |
| Sample/Concentra tion of Comp. Ex. | 1 μM | 5 μM | 10 Mm | 20 μM | 80 μM |
| Comp. Ex. (Dexamethasone) | 22.64±1. 27 | 71.72±0. 69 | 76.82±1. 73 | 81.78±0. 60 | 84.23±0. 41 |

As shown in Table 10, the COX-2 mRNA expression inhibition rate of the glycerol glucoside-based compound sample (EPS-S) obtained in the present Example was -9.09% at 10 ppm, 74.44% at 100 ppm, 76.43° at 500 ppm, and 83.19% at 1000 ppm.

On the other hand, it was found that the COX-2 mRNA expression inhibition rate of dexamethasone (the positive control group) was 22.64% at 1 μM, 71.72% at 5 μM, 76.82% at 10 μM, 81.78% at 20 μM, and 84.23% at 80 μM.

**2-7. Analysis of nitric oxide ($NO_2$) production amount and inhibition ability (Non-enzymatic Method)**

An NO assay was performed to evaluate an amount of nitric oxide ($NO_2$) production and a production inhibition ability of the glycerol glucoside-based compound sample (EPS-S).

Specifically, subcultured RAW264.7 cells were aliquoted into a 96-well plate at $1\times10^5$ cells/well, and cultured at 37°C in an incubator containing 5% carbon dioxide for 24 hours, and then a culture media was replaced with a serum-free media. Then, each sample was diluted by concentration, treated onto the cells with LPS (final 1 μg/mL), and incubated for 24 hours; then, after reacting the cell culture solution with a griess reagent, an absorbance was measured at 540 nm with a microplate reader, and an $NO_2$ production amount was obtained by substituting the absorbance into a standard nitric oxide solution calibration curve; and an $NO_2$ production inhibition rate (%) was analyzed through Equation 2 and expressed as a mean ± standard deviation. Dexamethasone was used as a positive control group.

[Table 11]

| | $NO_2$ production mean (μM) ± standard deviation | $NO_2$ production inhibition rate (%) ± standard deviation |
|---|---|---|
| Untreated group | 9.23±0.00 | 100±0.00 |
| Negative control | 24.59±0.43 | 0±0.00 |

[Table 12]

| $NO_2$ production amount (μM ) ± standard deviation for each concentration of sample | | | |
|---|---|---|---|
| Sample/Concentration of Ex. | 10 ppm | 100 ppm | 1000 ppm |
| Ex. (EPS-S) | 23.14±0.25 | 22.57±0.16 | 20.90±0.50 |
| Sample/Concentration of Comp. Ex. | 10 μM | 20 μM | 80 μM |
| Comp. Ex. (Dexamethasone) | 21.29±0.59 | 20.79±0.12 | 19.62±0.50 |

[Table 13]

| $NO_2$ production inhibition rate (%) ± standard deviation for each concentration of sample | | | |
|---|---|---|---|
| Sample/Concentration of Ex. | 100 ppm | 500 ppm | 1000 ppm |
| Ex. (EPS-S) | 9.47±1.60 | 13.16±1.06 | 24.02±3.27 |
| Sample/Concentration of Comp. Ex. | 10 μM | 20 μM | 80 μM |
| Comp. Ex. (Dexamethasone) | 21.48±3.82 | 24.71±0.80 | 32.33±3.27 |

As shown in Tables 12 and 13 above, the $NO_2$ production amount of the glycerol glucoside-based compound

sample (EPS-S) obtained in the present Example was 23.14 μM at 100 ppm, 22.57 μM at 500 ppm, and 20.90 μM at 1000 ppm, and the $NO_2$ production inhibition rate was 9.47% at 100 ppm, 13.16% at 500 ppm, and 24.02% at 1000 ppm, respectively.

On the other hand, the $NO_2$ production amount of dexamethasone (the positive control group) was 21.29 μM at 10 μM, 20.79 μM at 20 μM, and 19.62 μM at 80 μM, and the $NO_2$ production inhibition rate was 21.48° at 10 μM, 24.71% at 20 μM, and 32.33% at 80 μM.

**2-8. Analysis of nitric oxide (total NO) production and inhibition ability (Enzymatic Method)**

An NO assay was performed to evaluate an amount of nitric oxide (total NO) production and a production inhibition rate of the glycerol glucoside-based compound sample (EPS-S).

Specifically, subcultured RAW264.7 cells were aliquoted into a 96-well plate at $1\times10^5$ cells/well, and cultured at 37°C in an incubator containing 5% carbon dioxide for 24 hours, and then a culture media was replaced with a serum-free media. Then, each sample is diluted by concentration and treated onto the cells with LPS (final 1 μg/mL). After culturing for 24 hours, a cell culture medium was treated with nitrate reductase, enzyme cofactor and enhancer to react. A Griess reagent was added and an absorbance was measured at 540 nm with a microplate reader. A total NO production was obtained by substituting the absorbance into a standard nitric oxide calibration curve, and a total NO production inhibition rate (%) was analyzed through Equation 2 and expressed as a mean ± standard deviation. Dexamethasone was used as a positive control group.

[Table 14]

| | Total NO production mean (μM) ± standard deviation | Total NO production inhibition rate (%) ± standard deviation |
|---|---|---|
| Untreated group | 18.99±0.16 | 100±0.00 |
| Negative control | 57.71±1.87 | 0±0.00 |

[Table 15]

| Total NO production (μM ) ± standard deviation for each concentration of the sample | | | |
|---|---|---|---|
| Sample/Concentration of Ex. | 100 ppm | 500 ppm | 1000 ppm |
| Ex. (EPS-S) | 62.07±0.48 | 59.20±0.86 | 53.38±0.18 |
| Sample/Concentration of Comp. Ex. | 10 μM | 20 μM | 80 μM |
| Comp. Ex. (Dexamethasone) | 41.15±0.43 | 38.84±1.24 | 37.82±0.75 |

[Table 16]

| Total NO production inhibition rate (%) ± standard deviation for each concentration of the sample | | | |
|---|---|---|---|
| Sample/Concentration of Ex. | 10 ppm | 100 ppm | 1000 ppm |
| Ex. (EPS-S) | -11.26±1.24 | -3.85±2.22 | 11.17±0.48 |
| Sample/Concentration of Comp. Ex. | 10 μM | 20 μM | 80 μM |
| Comp. Ex. (Dexamethasone) | 42.77±1.10 | 48.72±3.21 | 51.37±1.95 |

As shown in Tables 15 and 16, the total NO production amount of the glycerol glucoside-based compound sample (EPS-S) obtained in the present example was 62.07 μM at 100 ppm, 59.20 μM at 500 ppm, and 53.38 μM at 1000 ppm, and the total NO production inhibition rate was -11.26% at 100 ppm, -3.84% at 500 ppm, and 11.17% at 1000 ppm, respectively.

On the other hand, the total NO production amount of dexamethasone (the positive control group) was 41.15 μM at 10 μM, 38.84 μM at 20 μM, and 37.82 μM at 80 μM, and the total NO production inhibition rate was 42.77% at 10 μM, 48.72% at 20 μM, and 51.37% at 80 μM.

**2-9. Analysis of expression of p-IKK, p-IxB and p-NF-κB proteins involved in inflammation-related signal pathways**

A western blot was performed as follows to observe p-IKK, p-IκB and p-NF-κB protein expression abilities of the glycerol glucoside-based compound sample (EPS-S).

Specifically, the subcultured mouse-derived macrophage line, RAW264.7 cells, were aliquoted in a 60 mm dish at $1.5\times10^6$ cells, and cultured for 24 hours at 37°C in an incubator containing 5% carbon dioxide, and then a culture media was replaced with a serum-free media. Then, the glycerol glucoside-based compound sample (EPS-S) was diluted by concentration and treated onto the cells with LPS (final 1 μg/mL). After culturing for 15 minutes, protein was lysed (lysis) to recover cytoplasmic and nuclear extracts, and proteins were separated by size through SDS-PAGE. After transferring the protein to a membrane through a transfer, a primary antibody was attached thereto and reacted at 4°C overnight. After the primary reaction, a secondary antibody was attached thereto, and reacted at room temperature for 1 hour, an ECL solution was sprayed on the entire surface, and a band image was obtained through a bioimage analyzer. After obtaining a band intensity of each band through Image J, normalization was performed using β-acitn and substituted in Equation 7 below to obtain the expression rate of p-IKK, p-IκB and p-NF-κB proteins in the treated sample group compared to the untreated group.

Here, FIG. 6 is a schematic diagram illustrating signal pathways of p-IKK, p-IκB and p-NF-κB, and FIG. 7 is a photograph illustrating protein bands of p-IKK, p-IκB and p-NF-κB.

[Equation 7]

$$Protein\ expression\ rate\ (\%) = \frac{Band\ intensity\ of\ treated\ sample}{Band\ intensity\ of\ untreated\ group} \times 100$$

[Table 17]

| Sample (Concentration) | Untreated | Negative control (1 mg/mL) | Ex. (EPS-S, 100 ppm) |
|---|---|---|---|
| p-IKK protein expression rate (%) | 100 | 1001.95 | 514.98 |
| p-IκB protein expression rate (%) | 100 | 390.06 | 203.52 |
| p-NF-κB protein expression rate (%) | 100 | 222.25 | 185.30 |

As shown in Table 17, expression rates of p-IKK, p-IκB and p-NF-κB proteins involved in the inflammation-related signal pathway by Western blot were examined, respectively.

Specifically, the expression rate of p-IKK protein was 1001.95% in the negative control group compared to 100% in the untreated group, and it was found to be 514.98% at 100 ppm of the glycerol glucoside-based compound sample (EPS-S). In addition, the expression rate of p-IκB protein was 390.06% in the negative control group compared to 100% in the untreated group, and it was found to be 203.52° at 100 ppm of the glycerol glucoside-based compound sample (EPS-S). Furthermore, the expression rate of p-NF-kB protein was 222.25% in the negative control group compared to 100% in the untreated group, and it was found to be 185.30% at 100 ppm of the glycerol glucoside-based compound sample (EPS-S).

**[Formulation Example 1: Cream Preparation]**

Using the glycerol glucoside-based compound extracted and fractionated from seaweed, a cream formulation that may be applied to the human body was prepared according to the composition shown in Table 18 below by a conventional method. In this case, in Table 18, the content unit of each component is percent by weight (wt%).

[Table 18]

| NO | INCI NAME | Actual Wt (%) | CAS No. |
|---|---|---|---|
| 1 | Water | 잔량 | 7732-18-5 |
| 2 | Propanediol | 7.0000000 | 504-63-2 |
| 3 | Glycerin | 5.0000000 | 56-81-5 |

(continued)

| NO | INCI NAME | Actual Wt (%) | CAS No. |
|---|---|---|---|
| 4 | Chlorella Vulgaris Extract | 3.0000000 | 223749-83-5 |
| 5 | Compound of Chemical Formula 3 | 0.01-5.0 | - |
| 6 | Polysorbate 20 | 0.6000000 | 9005-64-5 (Generic) |
| 7 | Glycereth-26 | 0.5000000 | 31694-55-0 (generic) |
| 8 | Dipropylene Glycol | 0.4400000 | 110-98-5 |
| 9 | PEG-60 Hydrogenated Castor Oil | 0.4000000 | 61788-85-0 |
| 10 | Hydroxyacetophenone | 0.3800000 | 99-93-4 |
| 11 | Caprylyl Glycol | 0.1790000 | 1117-86-8 |
| 12 | 1,2-Hexanediol | 0.0540000 | 6920-22-5 |
| 13 | Ethylhexylglycerin | 0.0300000 | 70445-33-9 |
| 14 | Disodium EDTA | 0.0200000 | 139-33-3 |
| 15 | Dipotassium Glycyrrhizate | 0.0010000 | 68797-35-3 |
| TOTAL | | 100.00000000 | |

**[Experimental Example 3] Clinical evaluation of cosmetics**

A clinical trial was conducted as follows for a cream formulation (EPS cream) containing a glycerol glucoside compound extracted and fractionated from seaweed.

A highly moisturizing cream formulation containing a glycerol glucoside compound was used as Sample A, and a commercially available highly moisturizing cream as a control was used as Sample B. In addition, total five women participated in this clinical evaluation, and the basic information and age distribution of the study subjects are shown in Table 19 below.

[Table 19]

| | |
|---|---|
| Registered study subjects (persons) | 5 people |
| Final completed study subjects (persons) | 5 people |
| Average age (standard deviation) | 46.20 (5.89) |
| Gender | Femail |
| Age | 40s (4 people) / 50s (1 person) |

**3-1. Skin texture evaluation**

A skin texture of a cheek area and a wrinkle area around eyes was measured using PRIMOS (Phaseshift Rapid In vivo Measurement Of Skin high resolution, GFMesstechnik GmbH, Germany).

Specifically, a subject's face was fixed to a specially designed PRIMOS measurement fixture set to prevent contraction and relaxation and movement of the measurement site. In order to measure the same area for every measurement, the equipment was fixed and measured to match the test site. The skin texture according to the application of the sample was analyzed using PRIMOS software (PRIMOS version 5.8E), and the three-dimensional measurement of the PRIMOS shows a change of parallel projection stripes projected on the skin according to a height difference of the skin surface, and the degree of such change was quantitatively calculated by a computer.

In such a case, as skin texture variable values through roughness analysis, there are Ra (Average roughness), Rq (Root mean square roughness), and Rmax (Maximum roughness depth), and when the value of each variable analyzed by roughness measurement decreases, it means that the skin texture is improved. In addition, a decrease rate of the roughness variable value by PRIMOS High Resolution was calculated using Equation 8 below.

[Equation 8]

$$Decrease\ rate\ (\%) = \frac{Measured\ value\ before\ sample\ application}{Measured\ value\ after\ sample\ application} \times 100$$

[Table 20]

| | Ex. (Sample A) | | Comp. Ex. (Sample B) | |
|---|---|---|---|---|
| | After 2 weeks of application | After 4 weeks of application | After 2 weeks of application | After 4 weeks of application |
| Ra | 2.618 | 9.028 | -1.499 | -0.315 |
| Rq | 2.882 | 8.994 | -0.971 | 0.085 |
| Rmax | 1.871 | 8.078 | 3.106 | 4.725 |

As shown in Table 20, as a result of analyzing the measured values for each roughness variable in the cheeks and eye area, in the case of the Ra value, the application site of the high moisturizing cream (Sample A) of Example showed a statistically significant ($p<0.05$) decrease after 4 weeks of application of the sample, compared to before application of the sample. On the other hand, it was found that the application site of the control sample B did not show a statistically significant difference after 2 weeks of application and after 4 weeks of application compared to before application of the sample.

**3-2. Skin elasticity evaluation**

A skin elasticity was evaluated in the same area of the cheeks of the study subjects using a Cutometer MPA580 (Courage and Khazaka, Germany).

Specifically, the Cutometer MPA580, a device for measuring elasticity, measures skin elasticity based on the principle that the skin returns to its original shape when the skin is sucked into a probe with a continuous negative pressure for the measurement time and then the negative pressure is removed; where a probe with a diameter of 2 mm connected to a device was attached to the skin and measured in a non-invasive manner. In this test, Mode 1 was used as the measurement condition, and measurement results were obtained by calculating an arithmetic average after measuring three consecutive times under the conditions of a constant negative pressure of 450 mbar, a suction time of 2 seconds, and a relaxation time of 2 seconds. Parameters related to skin elasticity are R2, R5, and R7, and as these elasticity measurement values (R2, R5, R7) increase, it means that the skin elasticity increases, and the skin elasticity increase rate was obtained using Equation 9.

[Equation 9]

$$Increase\ rate\ (\%) = \frac{Measured\ value\ after\ sample\ application}{Measured\ value\ before\ sample\ application} \times 100$$

[Table 21]

| | Ex. (Sample A) | | Comp. Ex. (Sample B) | |
|---|---|---|---|---|
| | After 2 weeks of application | After 4 weeks of application | After 2 weeks of application | After 4 weeks of application |
| R2 | 2.399 | 3.355 | 0.415 | 1.013 |
| R5 | 7.968 | 16.269 | 1.678 | 0.708 |
| R7 | 9.131 | 15.333 | 3.758 | 0.980 |

As shown in Table 21 above, as a result of analyzing the measured values for each skin elasticity variable in

the cheek area, it was appreciated that the application site of the high moisturizing cream (Sample A) of Example showed a statistically significant level ($p<0.05$) of decrease in R2, R5, and R7 values after 2 weeks of application and after 4 weeks of application compared to before application of the sample.

In addition, the application site of the high moisturizing cream (Sample A) of Example showed a statistically significant level ($p<0.05$) of increase in the R2 value after 2 weeks of application and a statistically significant level ($p<0.05$) of increase in the R5 and R7 values after 4 weeks of application, compared to before application of the sample.

## Claims

1. A compound represented by the following Chemical Formula 1, or a pharmaceutically acceptable salt, or a solvate thereof:

[Chemical Formula 1]

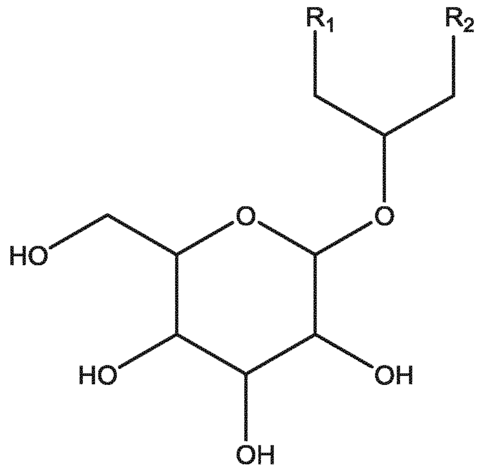

wherein in the above Chemical Formula,
$R_1$ and $R_2$ are the same as or different from each other, each independently being selected from: a hydroxyl group and a $C_1$ to $C_6$ alkoxy group, wherein at least one of $R_1$ and $R_2$ is a $C_1$ to $C_6$ alkoxy group.

2. The compound of claim 1, or the pharmaceutically acceptable salt, or the solvate thereof, wherein each of $R_1$, $R_2$, or $R_1$ and $R_2$ is a methoxy group.

3. The compound of claim 1, or the pharmaceutically acceptable salt, or the solvate thereof, wherein the compound represented by the above Chemical Formula 1 is represented by Chemical Formula 2 or Chemical Formula 3.

[Chemical Formula 2]

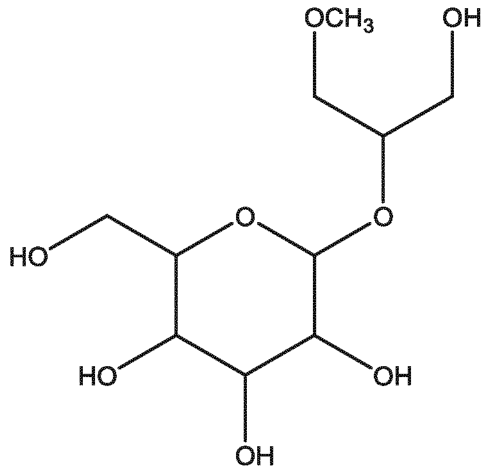

[Chemical Formula 3]

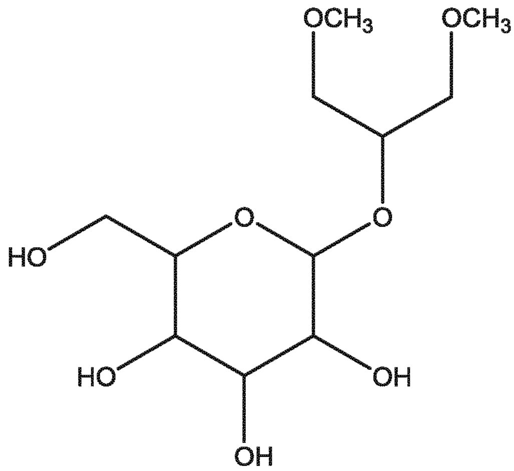

4. A pharmaceutical composition for UV shield or anti-inflammation, comprising the compound, or the pharmaceutically acceptable salt, or the solvate thereof according to any one of claims 1 to 3.

5. A cosmetic composition for UV shield or anti-inflammation, comprising the compound, or the pharmaceutically acceptable salt, or the solvate thereof according to any one of claims 1 to 3.

6. A food composition for UV shield or anti-inflammation, comprising the compound, or the pharmaceutically acceptable salt, or the solvate thereof according to any one of claims 1 to 3.

7. The food composition of claim 6, wherein the composition is a functional food, a health supplement or a health functional food.

FIG. 1

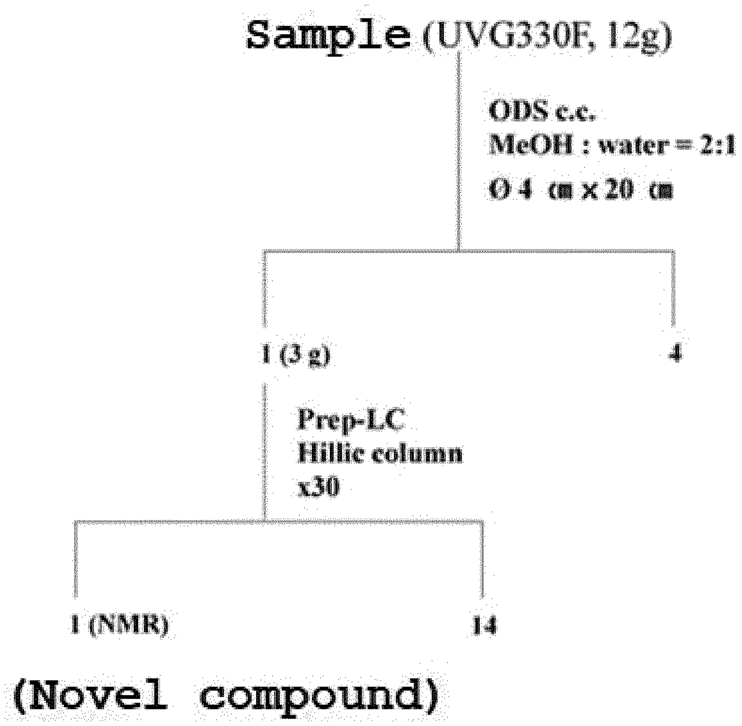
Sample (UVG330F, 12g)
ODS c.c.
MeOH : water = 2:1
Ø 4 cm x 20 cm
1 (3 g)
4
Prep-LC
Hillic column
x30
1 (NMR)
14
(Novel compound)

FIG. 2

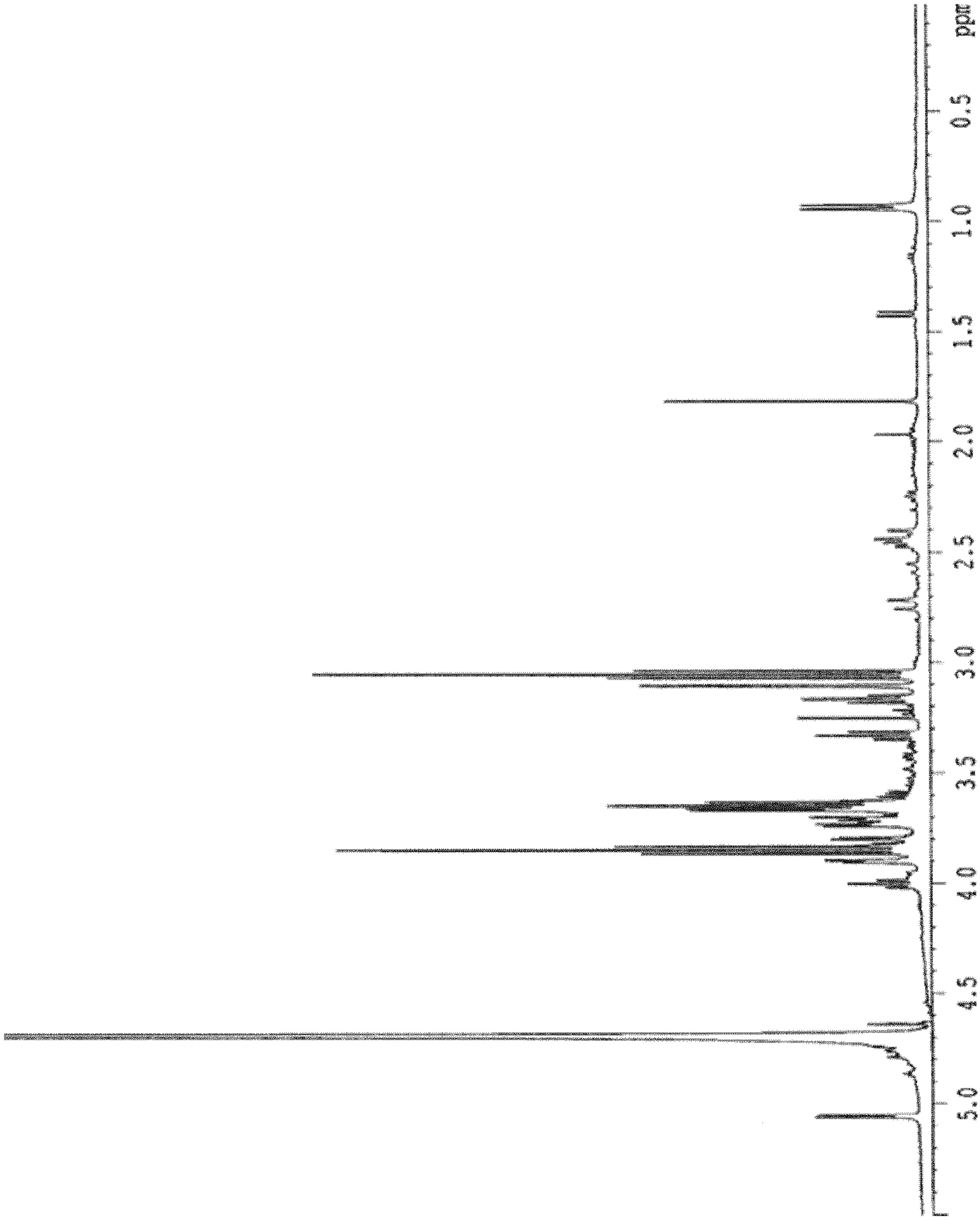

FIG. 3

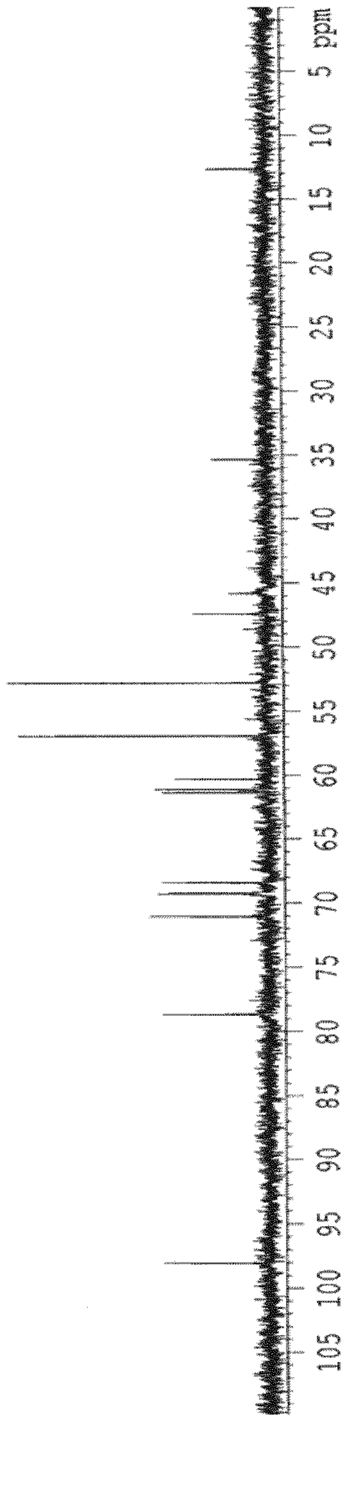

FIG. 4

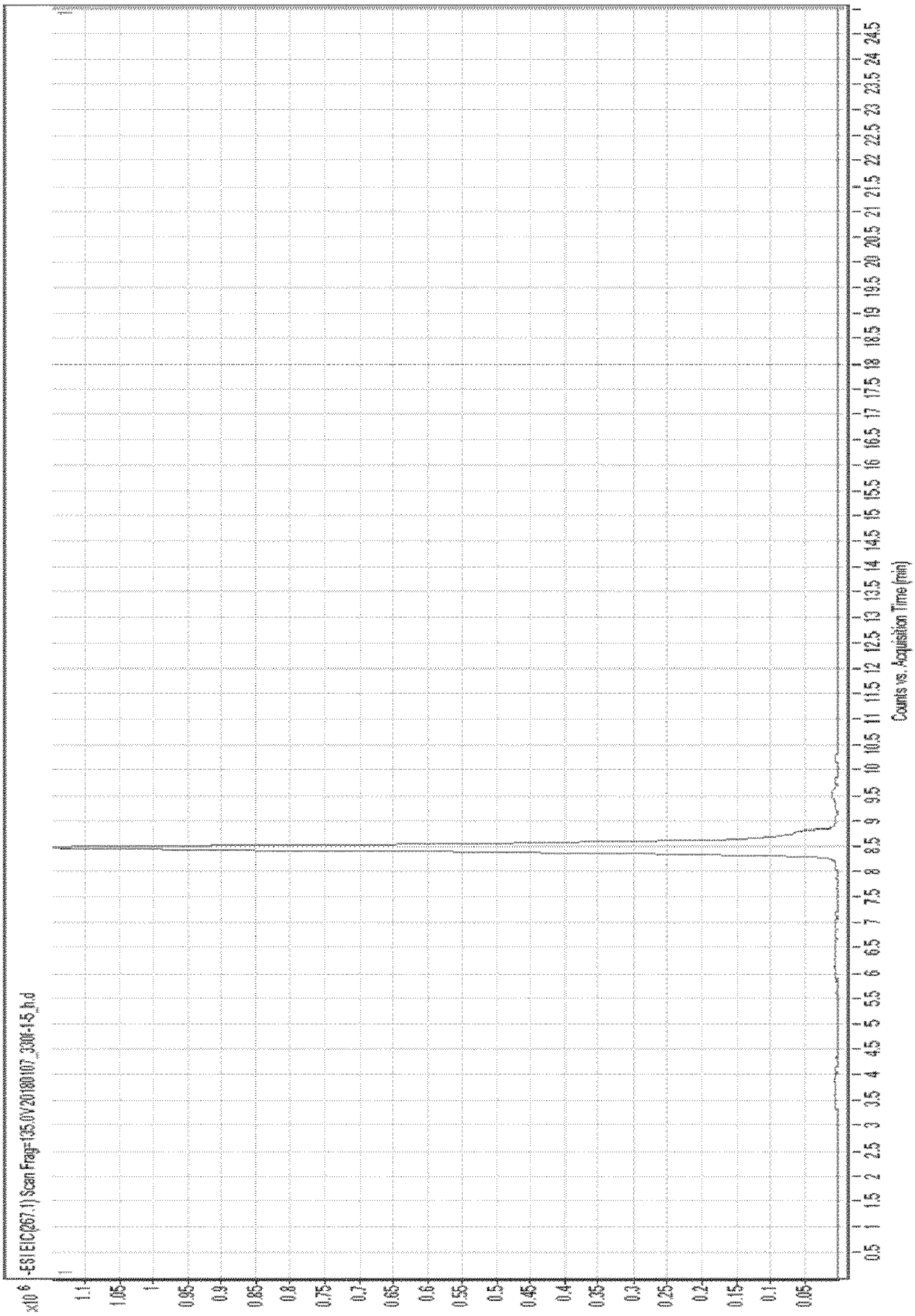
-ESI EIC(267.1) Scan Frag=135.0V 20180107_300K-1-5_h.d
x10^6
Counts vs. Acquisition Time (min)

FIG. 5

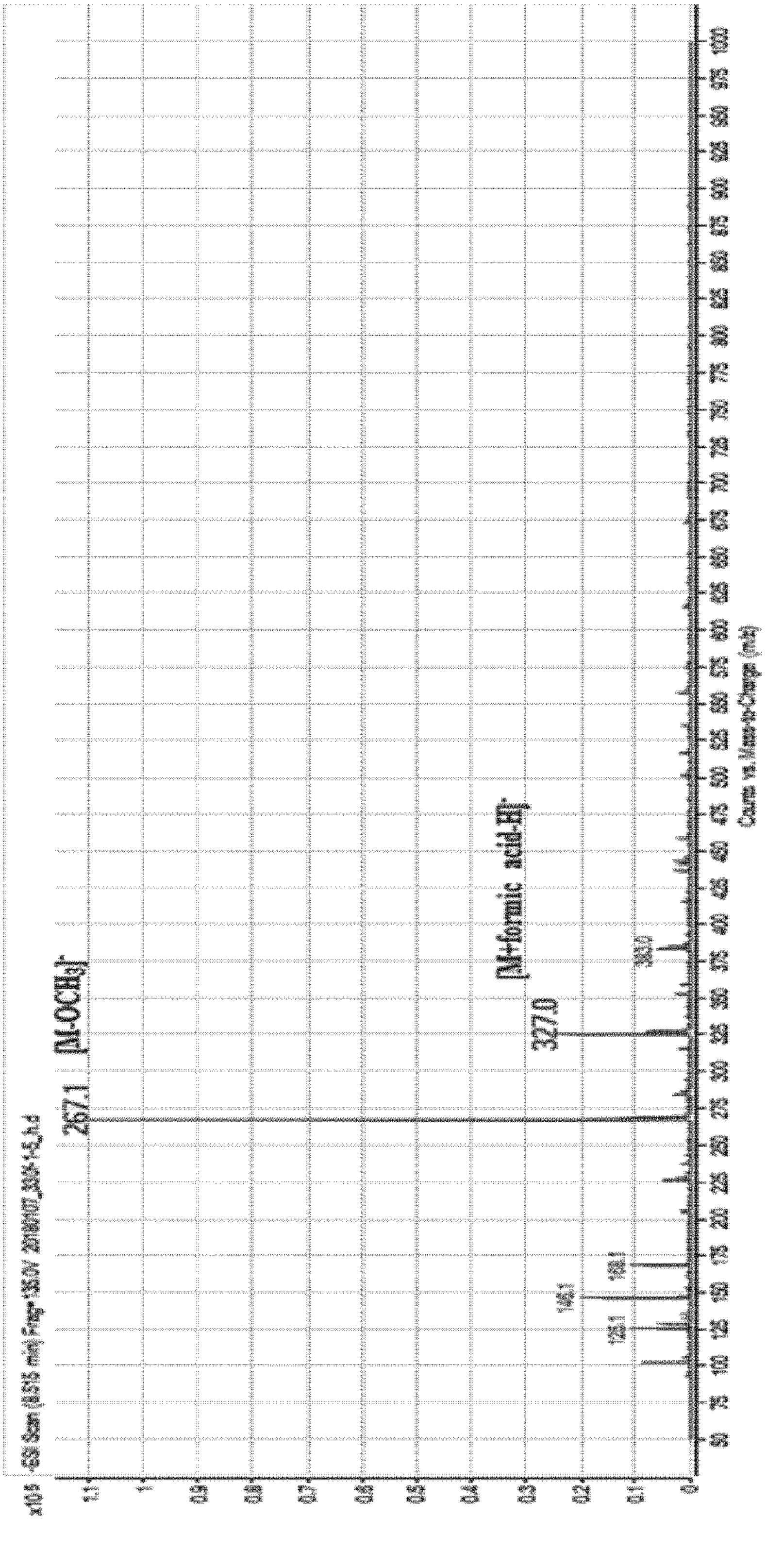

FIG. 6

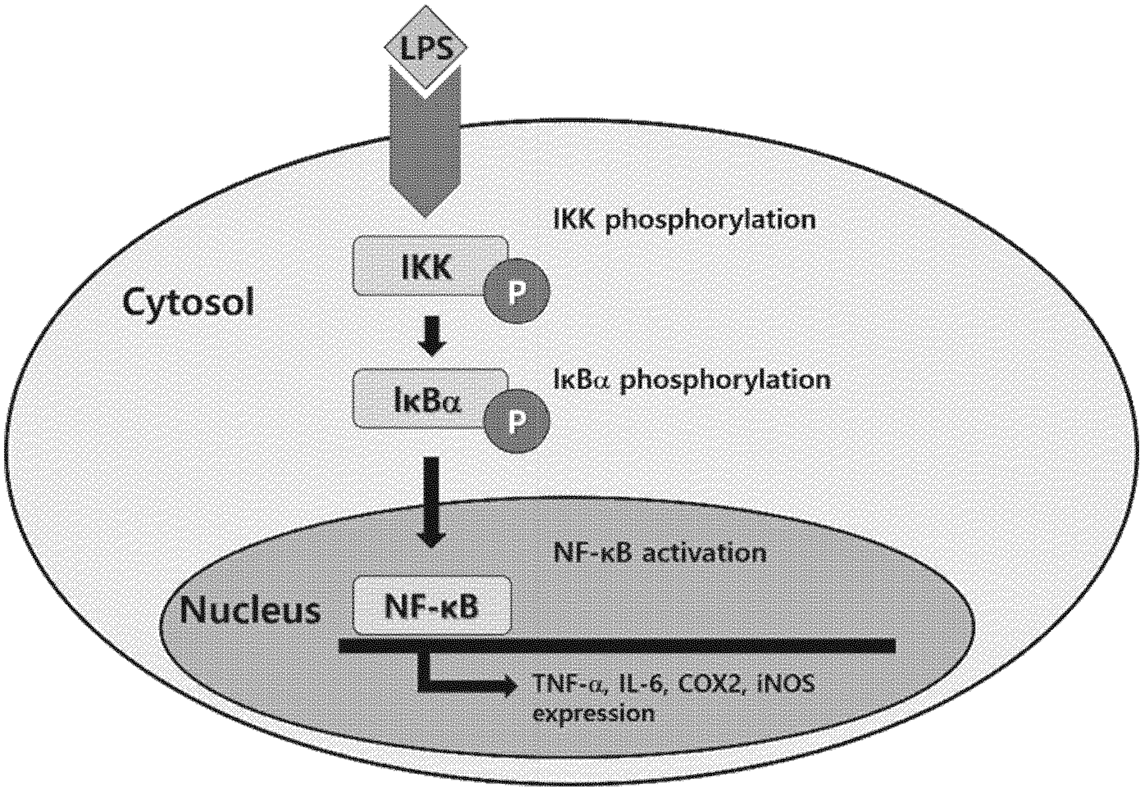
LPS
IKK phosphorylation
IKK
P
Cytosol
IκBα
P
IκBα phosphorylation
NF-κB activation
Nucleus
NF-κB
TNF-α, IL-6, COX2, iNOS
expression

FIG. 7

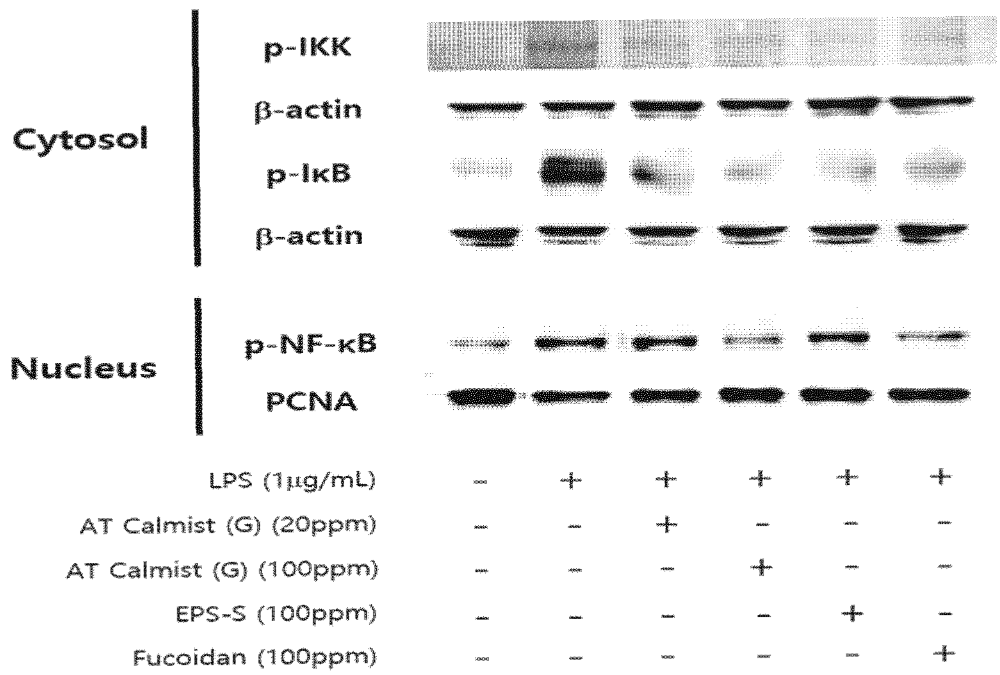
Cytosol
p-IKK
β-actin
p-IκB
β-actin
Nucleus
p-NF-κB
PCNA
LPS (1μg/mL) - + + + + +
AT Calmist (G) (20ppm) - - + - - -
AT Calmist (G) (100ppm) - - - + - -
EPS-S (100ppm) - - - - + -
Fucoidan (100ppm) - - - - - +

## INTERNATIONAL SEARCH REPORT

International application No.
**PCT/KR2021/003463**

### A. CLASSIFICATION OF SUBJECT MATTER

**C07H 15/04**(2006.01)i; **A61K 31/7028**(2006.01)i; **A61P 17/00**(2006.01)i; **A61P 17/16**(2006.01)i; **A61P 29/00**(2006.01)i; **A61K 8/60**(2006.01)i; **A61Q 17/04**(2006.01)i; **A61Q 19/08**(2006.01)i; **A23L 33/10**(2016.01)i

According to International Patent Classification (IPC) or to both national classification and IPC

### B. FIELDS SEARCHED

Minimum documentation searched (classification system followed by classification symbols)

C07H 15/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 글리세롤 글루코시드(glycerol glucoside), 자외선 차단(ultraviolet block), 항염(anti-inflammatory)

### C. DOCUMENTS CONSIDERED TO BE RELEVANT

| Category* | Citation of document, with indication, where appropriate, of the relevant passages | Relevant to claim No. |
|---|---|---|
| X<br>A | RENIRIE, R. et al. Regio- and stereoselective glucosylation of diols by sucrose phosphorylase using sucrose or glucose 1-phosphate as glucosyl donor. Journal of molecular catalysis B: Enzymatic. 2010, vol. 67, nos. 3-4, pp. 219-224.<br>See abstract; and figure 3. | 1-3<br>4-7 |
| X | LULEY-GOEDL, C. et al. Regioselective O-glucosylation by sucrose phosphorylase: a promising route for functional diversification of a range of 1, 2-propanediols. Carbohydrate research. 2010, vol. 345, no. 1 2, pp. 1736-1740.<br>See abstract; formula 1 (compound 2a). | 1-3 |
| A | KR 10-2019-0081731 A (ATHENA CO., LTD.) 09 July 2019 (2019-07-09)<br>See claim 1; and figure 1. | 1-7 |
| A | JP 2017-132725 A (MIKIMOTO SEIYAKU KK) 03 August 2017 (2017-08-03)<br>See claim 3. | 1-7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 July 2021** | **19 July 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2021/003463

C. DOCUMENTS CONSIDERED TO BE RELEVANT

| Category* | Citation of document, with indication, where appropriate, of the relevant passages | Relevant to claim No. |
|---|---|---|
| A | COLOMBO, D. et al. Inhibitory effect of stabilized analogues of glycoglycerolipids on Epstein-Barr virus activation and mouse skin tumor promotion. Cancer letters. 2002, vol. 186, no. 1, pp. 37-41. See entire document. | 1-7 |
| A | COMPOSTELLA, F. et al. Synthesis of isosteric analogues of acylglycosylglycerols active as chemoprevention agents. European journal of organic chemistry. 2002, vol. 2002, no. 8, pp. 1429-1435. See entire document. | 1-7 |

Form PCT/ISA/210 (second sheet) (July 2019)

**INTERNATIONAL SEARCH REPORT**
**Information on patent family members**

International application No.
**PCT/KR2021/003463**

| Patent document cited in search report | | | Publication date (day/month/year) | Patent family member(s) | | | Publication date (day/month/year) |
|---|---|---|---|---|---|---|---|
| KR | 10-2019-0081731 | A | 09 July 2019 | WO | 2019-132621 | A1 | 04 July 2019 |
| JP | 2017-132725 | A | 03 August 2017 | JP | 6795306 | B2 | 02 December 2020 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **S.M. BERGE et al.** *J. Parmaceutical Sciences,* 1977, vol. 66 (1